# EUROPEAN PATENT SPECIFICATION

(11) **EP 2 227 966 B1**
(45) Date of publication and mention of the grant of the patent: **27.07.2016**
(21) Application number: 09173129.9
(22) Date of filing: 15.10.2009
(51) Int. Cl.: A23D 7/005, A23L 33/185, A23L 33/10, A23L 27/60

(54) **Condiment**
Würzmittel
Condiment

(30) Priority: 25.02.2009 EP 09153677
(43) Date of publication of application: 15.09.2010
(73) Proprietor: Coöperatie Avebe U.A., 9641 GK Veendam (NL)
(72) Inventor: Giuseppin, Marco Luigi Federico, 9461 JB Gieten (NL); Nieuwenhuijzen, Nelly Hermina, 9752 EE Haren (NL); Tromp, Robert Hans, 3524 CN Utrecht (NL)
(74) Representative: V.O.

(56) References cited:
- EP-A1- 1 867 323
- EP-A1- 1 920 662
- WO-A1-01/17541
- WO-A1-2004/105520
- WO-A1-2008/069649
- WO-A2-03/092603
- WO-A2-2004/002240
- GB-A- 2 356 790
- US-A- 4 129 663
- US-A1- 2002 119 915
- US-A1- 2006 035 827
- US-A1- 2008 181 990
- RALET M-C ET AL: "FRACTIONATION OF POTATO PROTEINS: SOLUBILITY, THERMAL COAGULATION AND EMULSIFYING PROPERTIES" LEBENSMITTEL WISSENSCHAFT UND TECHNOLOGIE, ACADEMIC PRESS, LONDON, GB, vol. 33, no. 5, 1 August 2000 (2000-08-01) , pages 380-387, XP001034682 ISSN: 0023-6438
- VAN KONINGSVELD GERRIT A ET AL: "Effects of protein composition and enzymatic activity on formation and properties of potato protein stabilized emulsions" JOURNAL OF AGRICULTURAL AND FOOD CHEMISTRY,, vol. 54, no. 17, 1 August 2006 (2006-08-01), pages 6419-6427, XP002553907
- LOKRA S ET AL: "Comparison of composition, enzyme activity and selected functional properties of potato proteins isolated from potato juice with two different expanded bed resins" LEBENSMITTEL WISSENSCHAFT UND TECHNOLOGIE, ACADEMIC PRESS, LONDON, GB, vol. 42, no. 4, 1 May 2009 (2009-05-01), pages 906-913, XP025885570 ISSN: 0023-6438 [retrieved on 2008-12-06]
- ANONYMOUS: 'Tate & Lyle launches flexible new wheat protein isolates' FOODNAVIGATOR.COM, [Online] 13 January 2006, XP055238739 Retrieved from the Internet: <URL:http://www.foodnavigator.com/content/v iew/print/29295> [retrieved on 2016-01-05]

## Description

The invention relates to a condiment and a method for preparing a condiment.

US 2008/0181990 relates to edible emulsions, in particular condiments, such as salad-dressings and spreads, comprising wheat protein isolate. Such condiments often contain egg-based ingredients, such as egg protein and/or egg yolk as an emulsifying ingredient in such emulsions, *inter alia* for possible medical problems associated with the consumption of egg constituents, such as an allergic reaction or problems associated with high cholesterol levels. It is mentioned that egg protein-based emulsifying agent in dressings and spreads may be replaced with other proteinaceous emulsifiers such as soy protein or whey protein. However, according to US 2008/0181990 these may not satisfactorily reproduce the texture and flavour associated with egg-containing dressings and spreads. Therefore, it is proposed to include wheat protein isolate in the emulsion. The examples all show the use of the wheat protein isolate in combination with another protein such as a soy protein (in soy bean powder, a milk protein (whey protein) and/or a polysaccharide, such as starch. Also egg-protein may be present.

However, a considerable part of the world's population may have a food-intolerance towards or be allergic for wheat protein (gluten), soy protein, milk protein or, as ready indicated above, egg protein. Therefore, there is a need for an alternative to the use of such emulsifying proteins. This need is also illustrated in GB2356790A, which describes an emulsifier obtained from carob germ and its use in fine food applications as a possible replacement of egg ingredients or milk protein derivatives.

In particular there is a need for such an alternative that is suitable to provide an edible emulsion that has a satisfactory texture and taste.

In particular, there is a need for such an emulsion in the form of dressing, a spread or another thick-viscous emulsion, that maintains a satisfactory viscosity for at least several weeks, preferably for more than a month.

Further, condiments comprising a substantial amount of egg-yolk as emulsifier, such as mayonnaise or dressings comprising egg-yolk, suffer from emulsion instability when frozen (and thawed again). Thus these condiments are not really suitable for use in frozen foods. There is therefore also need for an alternative condiment that has an improved freeze-thaw stability.

It is an objective of the present invention to address one or more of these needs. One or more other objectives that may be addressed are apparent from the description below.

It has now been found possible to provide a condiment that meets one or more of the objectives underlying the invention, by including a specific protein fraction of a specific plant material.

Accordingly, the present invention relates to a condiment comprising an emulsion of an aqueous phase and an oil or fat phase, the emulsion comprising a potato protein protease inhibitor isolate, wherein the condiment further comprises at least one ingredient selected from the group of thickening agents, food acids, flavourings, preservatives, anti-oxidants and colourings.

Potato protein protease inhibitor isolate is in principle known in the art. US 2002/0119915 A1 describes a nutritional intervention composition in powder form comprising an extract of potato containing proteinase inhibitor. Emulsification properties of certain potato protein fractions are described in J. Agaric. Food Chem. 2006, 54, 6419-6427 and also in Lebensmittel Wissenschaft und technologie, academic press, Londong, GB, vol. 33, no. 5, 1 August 2000, pages 380-387.

The condiment may in particular be selected from the group of spreads and dressings, in particular from the group of mayonnaises, mayonnaise-substitutes, salad dressings and Hollandaise sauce, more in particular from the group of mayonnaises and mayonnaise-substitutes.

A condiment according to the invention has a satisfactory texture and satisfactory organoleptic properties. It has in particular been found that it is possible to provide a condiment that does not have an unacceptable astringent taste, which is surprising as a solution of potato protein protease inhibitor isolate in water (e.g. 1 %) does tend to taste astringent.

It is an advantage of the invention that it is possible to provide a condiment with a specific viscosity at a relatively low concentration of protein, compared to reference condiments based on at least some other proteins, e.g. on egg protein. In particular, this may be beneficial in that no additional thickening agent or less additional thickening agent (such as starch) is required to obtain a product with a specific viscosity. A reduced thickening agent concentration may be beneficial with respect to organoleptic properties, taste or nutritional value (*e.g*. starch adds to the caloric value of the product, but is generally considered an inferior food source than proteins, as the latter provide essential nutrients (amino acids) for an organism).

It has been found possible to provide a condiment with satisfactory properties, such as a satisfactory viscosity-stability and/or a satisfactory emulsion stability, and/or a satisfactory freeze-thaw stability also in the absence of traditionally used emulsifiers, such as egg yolk, artificial emulsifiers, such as chemically derivatised polysaccharide-type emulsifiers (e.g. starch-octenylsuccinate). Also, it has been found possible to provide a condiment with satisfactory properties, such as a satisfactory viscosity-stability and/or a satisfactory emulsion stability and/or a satisfactory freeze-thaw stability, without needing added mono- or diglycerides, which are often used in conventional food-emulsion to improve a property of the emulsion type emulsifiers.

Viscosity-stability, as used herein can be determined by measuring the viscosity as measured with a viscometer (type Brookfield DV-I+, Elscolab, the Netherlands) at a temperature of 5°C with spindle S93 and a speed of 10 rpm at at least two different points in time after preparation of the condiment, *e.g.* a first time within a day after preparation (after a night of storage) and a second time three weeks or six weeks after preparation. The lower the difference in viscosity of the condiment at the different points in time, the better the visocisity-stability. As will be understood by the skilled person, storage conditions, in particular storage temperature, may affect the viscosity-stability. Unless specified otherwise, when referred herein to viscosity-stability at 4 °C is meant. As will also be understood by the skilled person, a maximum allowable change in viscosity over a certain period of time is dependent on the type of product (type of condiment, desired product quality). Usually, a change in viscosity (when stored at 4 °C) of 15 % or less in the period of three weeks is considered acceptable. In particular, it has been found possible in accordance with the invention to provide a condiment of which the viscosity changes less than 10 %, or even less than 5 %, over a storage period of three weeks, at least for some embodiments, also in the absence of other emulsifiers than the potato protein protease inhibitor isolate.

Freeze-thaw stability, as used herein is, included stability of the emulsion after freezing and thawing (freeze-thaw emulsion stability), stability of the structure of the product in general and stability of the consistency of the product in general.

Freeze-thaw stability is determined by subjecting a product to the following freeze-thaw cycle: storing a (non-frozen) product in a freezer over night (at least 12 hours, at -22 °C), thawing the product at room temperature (20 °C) and visually evaluating product. Emulsion stability is evaluated by evaluating the surface of the product (with the naked eye) for the presence of phase separation. This allows assessment of how stable the emulsion is when exposed to freezing and thawing. The phase separation may manifest itself as oil or water droplets on the surface of the product or as an oil or water layer on the surface of the product. The more often the product can be frozen en thawed again without occurrence of phase separation, the higher its freeze-thaw emulsion stability. A product that is suitable for use after freezing and thawing should also at least substantially maintain a desired structure and consistency. Therefore, when establishing the freeze-thaw stability in general the product is also evaluated for changes in the structure and consistency of the product. This can also be done visually with the naked eye by a skilled person.

A product is considered freeze-thaw emulsion stable if it can be frozen and thawed again at least once, in particular at least twice, preferably at least three times, more preferably at least five times, without showing phase separation. It is considered freeze-thaw stable (in general) if the product further does not show significant (unacceptable) change in the structure and consistency, after one, in particular two, preferably three, more preferably five of freeze-thaw cycles.

The invention further is advantageous in that the pH of the product may be chosen within a wide range. In particular, the pH may be in the range of 2.5-6.5, more in particular in the range of 3.2-4.2, whilst demonstrating satisfactory viscosity-stability. As used herein, the pH is the pH of the water phase of the condiment as measured by a pH meter (WTW Inolab, type CR-QI 10101) at 20 °C.

In particular, the invention allows the provision of a condiment wherein one or more specific allergens are absent, or present in a relatively low concentration. In particular, the condiment may be essentially free of one or more of the following compounds: gluten, egg proteins, dairy proteins, egg-yolk and soy proteins. Good results have been obtained with a condiment that is essentially free of all other proteins than proteins from potato.

If desired, the condiment can be free of ingredients originating from an animal, and thereby suitable for a vegetarian or vegan diet.

From studies under conditions emulating conditions in the gastro-intestinal tract, including addition of gastric juice and pancreatic solution to an oil in water emulsion comprising the potato protein protease inhibitor, in particular in a coacervate with a hydrocolloid, it is contemplated that potato protein protease inhibitor in a condiment or other food may contribute to delay the digestion of the oil or fat phase in the stomach and small intestine. It is contemplated that the oil or fat phase or at least a significant part thereof may pass undigested through the first part of the digestive tract (duodenum and jejunum) into the final part of the small intestine, the ileum. Undigested triglycerides (solid fat and liquid oils in the oil or fat phase) in the ileum are recognised as a signal that the body is satisfied. The brain will therefore be informed that there is no need to take in more food. This signalling mechanism, also referred to as the 'Ileal brake mechanism', helps to reduce intake of food, and thereby may be used for preventing, helping to prevent or treating overweight.

Accordingly, potato protein protease inhibitor present in the condiment according to the invention, in particular when present as a coacervate of a potato protein protease inhibitor and a hydrocolloid, may helps to prevent or treat overweight.

In particular, potato protein protease inhibitor present in the condiment according to the invention, in particular when present as a coacervate of a potato protein protease inhibitor and a hydrocolloid, may trigger or contribute to the triggering of the Ileal brake mechanism.

The potato protein protease inhibitor, in particular a coacervate thereof, is amongst others advantageous in that it appears to be suitable, e.g. for stabilization of oil during gastro-intestinal passage, possibly up to the ileum, without needing to encapsulate the potato protein protease inhibitor or coacervate thereof in a crosslinked or non-crosslinked encapsulating material (see Example 3 'Stability of coacervates in simulated physiological model').

Accordingly, although in principle the potato protein protease inhibitor or coacervate thereof for use in preventing, helping to prevent or treating overweight may be encapsulated, in a preferred embodiment it is not encapsulated in an encapsulating material, and in particular not in a crosslinked encapsulating material.

Regarding condiments of the invention in which the potato protein protease inhibitor or coacervate forms part of an emulsion, in general, it will be understood by the skilled person that the potato protein protease inhibitor or coacervate will generally not be encapsulated in a crosslinked or non-crosslinked encapsulating material because of the nature of emulsions (emulsions being a multiphase system of two or more fluids, rather than of a fluid and a solid particulate material).

Subject to be treated in accordance with this aspect may in particular be selected from the group of humans and other mammals.

Advantageously, the potato protein protease inhibitor isolate contributes to an improved freeze-thaw stability, *i.e.* shows no visible phase-separation of the oil phase and the aqueous phase after freezing and thawing again, or at least less phase-separation compared to a prior art condiment, such as mayonnaise. Accordingly, in an advantageous embodiment, the condiment is a freeze-thaw stable condiment, *i.e.* a condiment that is essentially free of any visible phase-separation of the oil phase and the aqueous phase after freezing and thawing again. In particular, advantageous with respect to freeze thaw emulsion stability is a condiment comprising potato protein protease inhibitor isolate and a hydrocolloid, in particular coacervate of potato protein protease inhibitor isolate and a hydrocolloid.

Protease inhibitors in potatoes can be divided into different groups based on their molecular weight. The different groups of protease inhibitors are identified as protease inhibitor I (molecular weight of about 39 kDa), carboxypeptidase inhibitor (molecular weight of about 4 100 Da), protease inhibitors IIa and **IIb** (molecular weight of about 20.7 kDa), and protease inhibitor A5 (molecular weight of about 26 kDa). The ratio of these different groups of protease inhibitors in the total potato protein depends on the potato variety.

The potato protein protease inhibitor isolate may be obtained in a manner known *per se.* Existing methods for isolating potato proteins and potato protein fractions include fractionation, ion exchange, gel permeation, ultrafiltration, affinity and mixed-mode chromatography and fractionation by heat coagulation. An example of a method for obtaining native potato protein isolate is described in EP 1 920 662.

Potato protein protease inhibitor isolate is in general a fraction of whole potato protein that is enriched in protein protease inhibitor. The presence of protein protease inhibitor isolate in a condiment of the invention can in particular be verified by an increased weight to weight ratio of protein protease inhibitor to patatin, compared to whole potato protein. Usually the weight to weight ratio of protein protease inhibitor to patatin is at least 80:20, preferably at least 85:15, more preferably 90:10.

The potato protein protease inhibitor isolate may in particular be a native potato protein protease inhibitor isolate. The term "native potato protein" is used in this application is meant to refer to the potato protein without any significant physical or (bio)chemical modification or inactivation, in particular denaturation.

In an advantageous embodiment an isolate is present that is obtainable by a process for obtaining a native potato protein protease inhibitor isolate, comprising
- optionally subjecting potato fruit juice to a flocculation by a divalent metal cation, *e.g.* at a pH of about 7- 9;
- centrifuging the flocculated potato fruit juice, thereby forming a supernatant;
- subjecting the supernatant to adsorption chromatography, in particular to mixed mode adsorption chromatography, operated at a pH of less than 11 and a temperature of 5 to 35 °C using an adsorbent capable of binding potato protein, thereby adsorbing the native potato protein to the adsorbent, which chromatography step can be carried out using various forms of adsorbents (which are known *per se)* using for example a fixed bed, an expanded bed or a membrane adsorber;
- eluting native potato protein protease inhibitor isolate from the adsorbent with an eluent.

The native potato protein protease inhibitor isolate may for instance be eluted at a pH of 6.0-9.5 or at a pH below 3.9, in which latter case the pH is preferably between 2.5 and 3.5.

In a specific embodiment the native potato protein protease inhibitor isolate is selected from the group consisting of a protease inhibitor I isolate, a carboxypeptidase inhibitor isolate, a protease inhibitor IIa and IIb isolate, and a protease inhibitor A5 isolate. Such isolates are obtainable by ion exchange, or gel permeation chromatography.

In a specific embodiment, the native potato protein protease inhibitor isolate has an isoelectric point above 5.5, preferably above 5.8, a molecular weight of below 35 kDa, preferably of 4-30 kDa. More specifically, the native potato protein protease inhibitor isolate may have an isoelectric point of 8 or more.

In a specific embodiment, the native potato protein protease inhibitor isolate has a glycoalkaloid concentration of less than 150 ppm.

In a specific embodiment, the native potato protein protease inhibitor isolate has a water solubility of more than 90 %, preferably of more than 95 % at a pH of 3.5 or at a pH of 9.5 and a temperature of 25 °C.

In a specific embodiment, the native potato protein protease inhibitor isolate has a protein content of more than 75 %, preferably of more than 80 %, more preferably of more than 90 %, and even more preferably more than 95 %.

In a specific embodiment, the native potato protein protease inhibitor isolate contains less than 10 ppm sulphite.

Further details about particularly suitable methodology to obtain the isolate are *e.g.* found in WO 08/069650.

In a specific embodiment, a partially hydrolysed potato protein protease inhibitor isolate is present. Partially hydrolysed protease inhibitor isolate can be obtained by enzymic hydrolysis at elevated temperatures, in particular at 50- 70 °C, with pepsin or alkaline proteases. Partially hydrolysed protease inhibitor isolate may provide enhanced solubility and improved emulsification properties.

The potato protein protease inhibitor isolate concentration may be chosen within wide limits. Usually the concentration is at least 0.1 wt. %, based on the total weight of the condiment, for providing a product with a satisfactory stability. Preferably, the concentration is at least 0.2 wt. %, at least 0.4 wt. % or at least 0.5 wt. %. The upper limit is not particularly critical; the concentration may be up to 5 wt. % or more than 5 wt. %. However, at a high concentration gelation may occur, which may cause adverse effects in viscosity or consistency. Therefore, the concentration is usually chosen below the gelation concentration, in case gelation is undesired. In general good properties of the product are obtained with a potato protein protease inhibitor isolate in a concentration of 4 wt. % or less, without needing other emulsifying compounds and/or without needing structurising proteins and/or without needing high concentrations of thickening agent. In a preferred embodiment, the potato protein protease inhibitor isolate is present in a concentration of 2 wt. % or less, in particular in a concentration of 1.5 wt. % or less, more in particular in a concentration of 1.0 wt. % or less.

The potato protein protease inhibitor protein(s) may be the only protein(s) in the condiment or one or more additional proteins may be present. Usually, the fraction of potato protein protease inhibitor relative to the total protein content in a product according to the invention is at least 40 wt. %, preferably at least about 50 wt. % in particular at least 75 wt. %, more in particular at least 85 wt. %. The fraction of potato protein protease inhibitor relative tot the total protein content in a product according to the invention may be up to 100 wt. %, 90 wt. % or less, 80 wt. % or less or 60 wt. % or less, depending on the application.

In particular, one or more other potato proteins may be present, e.g. residual patatin that has not been removed from the isolate during fractionation of whole potato protein used for the preparation of the isolate. The concentration of potato protein protease inhibitor protein(s), as a total percentage of the potato protein content, usually is 75-100 wt. %, preferably at least 85 wt. %, in particular at least 90 wt. %. This is considered in particularly favourable for good rheological properties, such as viscosity-stability.

In a specific embodiment one or more other proteins are present as a coacervate with the potato protein protease inhibitor(s). A preferred example of such protein is gelatine, which may be of animal origin or prepared via a microbiological technique, using heterologous expression in a yeast or other microorganism. In particular in such embodiment, the concentration of said other protein(s) can be relatively high, *e.g.* in a concentration whereby the weight to weight ratio of said other protein to potato protein protease inhibitor(s), is in the range of 25:75 to 75: 25, in particular in the range of 60:40 to 40:60, more in particular about 50:50.

In principle, one or more other proteins may be present, such as one or more proteins selected from the group consisting of whey protein, soy protein, whole soybean protein, milk protein, safflower protein, canola protein, an egg protein, isolates of any thereof. However, good results have been obtained in the absence of any of these proteins, in particular with respect to viscosity-stability and/or with emulsion stability under simulated gastro-intestinal conditions. Amongst others in view of potential food intolerance of allergy issues, a specifically preferred product of the invention is free of these proteins. If present, the weight to weight ratio of the total of these proteins to the protein protease inhibitor(s) typically is less than 50:50, in particular 25:75 or less, more in particular 10:90 or less.

Preferably, at least a part of the potato protein protease inhibitor is present as a coacervate of the potato protein protease inhibitor and a hydrocolloid. The hydrocolloid may be neutral or be charged. In a specific embodiment the net charge of the hydrocolloid is opposite to the net charge of the potato protein protease inhibitor, in which case they can form a complex coacervate. In acidic products, this usually means that the hydrocolloid has a negative net charge. Acidic polysaccharides are preferred examples of hydrocolloids that can have a negative net charge (*e.g.* due to the presence of carboxyl groups, phosphate groups *etc.*). As mentioned above, gelatine may be present as a coacervate. In a further preferred embodiment, at least one hydrocolloid is present selected from the group of pectins (methoxyl-pectin, which may be high-methoxyl pectin or low-methoxyl pectin; amidated pectin or any other variation of pectin) arabic gum and alginates.

Further examples of hydrocolloids, include xanthan gum; sulphated polysaccharides, such as carrageenan; and carboxylated polysaccharides, for instance carboxyalkyl cellulose, *e.g.* carboxymethyl cellulose.

As mentioned above, a hydrocolloid may contribute to an improved freeze-thaw stability, in combination with the potato protein protease inhibitor isolate. Pectin, in particular methylated pectin, has been found a particular suitable hydrocolloid, *inter alia* to that purpose, contributing not only to an improved stability of the emulsion but also of the structure and consistency of a product according to the invention. Further, of xanthan gum it has been found that it is suitable to improve the freeze-thaw emulsion stability. It is envisaged that in particular other charged or uncharged polysaccharides, more in particular other anionic polysaccharides, such as alginate or carboxymethyl cellulose, may also have a positive effect on freeze-thaw stability in combination with the potato protein protease inhibitor isolate.

In case the isolate is present as a coacervate the weight to weight ratio of potato protein protease inhibitor isolate to hydrocolloid, usually is in the range of 25:75 to 75: 25, in particular in the range of 67:33 to 33:67, more in particular in the range of 60:40 to 40:60, *e.g.* about 50:50.

A product according to the invention further comprises an oil or fat phase. The oil or fat phase may be any edible liquid oil, any edible solid fat or mixture thereof. Preferably the oil or fat phase is of a vegetable origin. For instance, the oil or fat phase may comprise an oil selected from the group of corn oil, soybean oil, canola oil, vegetable oil, safflower oil, sunflower oil, nasturtium seed oil, mustard seed oil, olive oil, sesame oil, peanut oil, cottonseed oil, rice bran oil, babassu nut oil, castor oil, palm oil, palm kernel oil, rapeseed oil, low erucic acid rapeseed oil, lupin oil, jatropha oil, coconut oil, flaxseed oil, evening primrose oil, jojoba oil, cocoa butter, a low trans-fatty acid oil, a diacylglycerol oil, fractions of any thereof, and mixtures of any thereof.

The concentration of the oil or fat phase may be chosen within wide limits. It is an advantage of the invention that it is suitable to provide low-fat, medium-fat and high-fat products with satisfactory properties. In general, the oil content is at least 3 wt. % based on the total weight of the product, in particular at least 15 wt. %, more in particular at least 25 wt. %. In a specific embodiment the oil content is 30 wt. % or more. Usually the oil content is 80 wt. % or less, based on the total weight, in particular 60 wt. % or less, more in particular 50 wt. % or less. In a specific embodiment, the oil content is 30 wt. % or less.

The condiment usually is a thick-viscous liquid, a paste or a gel. The viscosity as measured with a viscometer (type Brookfield DV-I+, Elscolab, the Netherlands) at a temperature of 5°C with spindle S93 and a speed of 10 rpm usually is 8000 cp or more, in particular 25000 or more , more in particular 35000 or more. The viscosity usually is 180000 cp or less, in particular 140000 cp or less, more particular in the range of 45000 cp or less. A preferred viscosity in particular depends on the type of condiment, e.g. for a salad dressing a lower viscosity may be desired than for mayonnaise or the like.

For providing a product with a specific viscosity several parameters can be adjusted, such as the concentration of the potato protein protease inhibitor isolate, the presence of one or more thickening agents, the presence of salt *etc..*

As a thickening agent in particular one or more polysaccharide-type thickening agents may be present, such as one or more polysaccharides selected from the group of starch, pectin, guar gum and xanthan gum.

The total concentration of thickening agent (potato protein protease inhibitor isolate excluded but hydrocolloid (including hydrocolloid in coacervate) included) usually is 0-12 wt. %, based on the total weight of the product.

Preferably the total concentration of thickening agent is 10 wt. % or less, in particular 7 wt. %or less, more in particular 5 wt. % or less, even more in particular 4 wt. % or less. If present, the total concentration of thickening agent usually is at least 0.25 wt. %, in particular at least 0.5 wt. %, more in particular at least 1.0 wt. %, even more in particular at least at least 2.0 wt. % or at least 2.5 wt. %.

Further, a product according to the invention comprises one or more additional ingredients, in particular at least one ingredient selected from the group of food acids, such as vinegar, or lemon juice; flavourings, such as mustard, a sweetener (*e.g.* sugar or artificial sweetener), salt, a spice; preservatives; anti-oxidants; and colourants. Suitable examples and concentrations for such ingredients are known in the art, for specific types of condiments. *E.g.* a mayonnaise or mayonnaise-substitute according to the invention in general comprises mustard and at least one acid selected from vinegar and lemon juice.

The invention further relates to a method for preparing a condiment according to the invention. In a method according to the invention an aqueous phase is prepared comprising the protein protease inhibitor isolate. The aqueous phase is thereafter dispersed in the oil or fat phase or the aqueous phase is dispersed in the oil or fat phase (depending on the type of condiment). The dispersing can be done in a manner known *per se, e.g.* using a colloid mill or in a manner as described in the above cited prior art.

Other ingredients may be added to the aqueous phase or the oil or fat phase prior to dispersing, or thereafter to the emulsion. The order of adding may in principle be based on known methodology for preparing a specific condiment.

In a specific embodiment, wherein use is made of a heating step and wherein a salt-containing condiment, such as a salt-containing dressing, is prepared, salt is added after subjecting the emulsion to a heating step.

If thickening agent (*e.g.* starch) is added, it is preferably added after formation of the emulsion, or simultaneously with the oil or fat phase to the aqueous phase comprising the potato protein protease inhibitor isolate.

If a thickening agent is added that needs to be hydrated in order to be effective (*e.g.* starch or another hydrocolloid thickening agent), acidulants (if added) are preferably added after the thickening agents have been hydrated to a sufficient extent.

It is an advantage of the invention that the potato protein protease inhibitor isolate allows dispersing of the aqueous phase in the oil or fat phase without needing to heat the mixture (above ambient temperature) during the preparation of the emulsion, although use may be made of a heating step, if desired.

A method according to the invention is usually carried out at a temperature in the range of 4-85 °C. It is possible to carry out all steps at essentially the same temperature, e.g. at ambient temperature (15-25 °C), below ambient temperature or above ambient temperature.

In a specific embodiment, the method involves subjecting the aqueous phase, the oil or fat phase, the emulsion or the final condiment to a heat treatment. The heat treatment may in particular involve subjecting at least one of the aqueous phase, the oil or fat phase, the emulsion and the final condiment to a temperature in the range of 50-85 °C, more in particular to a temperature in the range of 65-85 °C or 75-85 °C. The duration of the treatment may be chosen within broad limits, e.g. in the range of 1-60 min, in particular in the range of 5-40 min.

A heat treatment may for instance be performed to alter the viscosity of the final product. It has in particular been found that a heat treatment of the aqueous phase comprising the potato protein protease inhibitor isolate has a viscosity increasing effect on the final product.

The invention will now be illustrated by the following examples.

### Examples

### Example 1: dressing comprising potato protein protease inhibitor isolate

Dressings (mayonnaise-substitute) were prepared with a Fryma colloid mill. The gap size was 1.45-1.50 mm. In total 6 kg of dressing was prepared per batch. Potato protein protease inhibitor isolate Solanic® 306p (PI) from Solanic a subsidiary of AVEBE, Foxhol, the Netherlands was used for the preparation of the dressing . The recipes of the dressings are given in Table 1. The protein was dissolved in the water phase and received a heat treatment as indicated in Table 1. The egg protein was supplied by Nive and contained minimally 30% protein.The protein content of the potato protein protease inhibitor was 90%. For the dressings in which Eliane SC 160 was used the lemon juice and the vinegar were added at the end. All ingredients except for the oil and the starch were added to the water phase in the Fryma. The starch was dispersed in the oil or fat phase. All ingredients were around 20°C at the start of the dressing preparation. The colloid mill was started and in around 1,5 minutes the oil was added to the mix. The mixing continued until a smooth and thick dressing could be observed. This took around 2-5 minutes. Next the dressing was filled into cups for further evaluation.

**Table 1. Dressing recipes**

| | 1 | 2 | 3 | 4 | 5 (ref) | 6 (ref) | 7 (ref) | 8 | 9 |
|---|---|---|---|---|---|---|---|---|---|
| pH | 3.4 | 3.6 | 3.7 | 3.4 | 4.0 | 4.0 | 3.5 | 3.4 | 3.4 |
| Oil (soy oil, Remia) | 30 | 50 | 30 | 50 | 50 | 30 | 50 | 50 | 50 |
| Eliane SC 160 (Avebe) | 4.5 | 1.75 | 4 | 1 | 2.5 | 5 | 1.75 | 1.75 | 1.75 |
| PI | 0.5 | 1 | 0.5 | 1 | | | | 1⁺ | 1 |
| Egg yolk powder (Nive) | | | | | 2.5 | 1.5 | | | |
| potato protein^{#} | | | | | | | 1 | | |
| Water | 52.95 | 35.2 | 53.45 | 35.95 | 32.95 | 51.45 | 35.2 | 35.2 | 35.2 |
| Mix* | 12.1 | 12.1 | 12.1 | 12.1 | 12.1 | 12.1 | 12.1 | 12.1 | 12.1 |
| Heat treatment (of water phase comprising PI) | No | No | Yes 30 min 80°C | Yes 30 min 80°C | No | No | No | No | No |

| | | | | | | | | | |
|---|---|---|---|---|---|---|---|---|---|
| *Mix containing salt (5%), Dyon mustard (16,6%), Sugar (24,9%), Vinegar (29,5%), lemon juice (23,2%) and K-sorbate (0,8%) # non-fractionated native potato protein isolate containing 50% patatin and 50% protease inhibitor (Solanic/AVEBE, The Netherlands) ⁺ powder was kept in a stove at 60 °C for 6 days prior to preparation of the dressing | | | | | | | | | |

### Texture measurements with Brookfield

The viscosity of the dressings was evaluated with a Brookfield (DV-I+, Elscolab, the Netherlands) with spindle S93 and a speed of 10 rpm.

The dressings were judged on taste, appearance, viscosity and stability over time. The viscosities of the dressings are given in Tables 2 and 3. The target viscosity (minimum) after 1 night in the fridge is 40.000 cp.

**Table 2. Viscosity of dressing , containing 50 % fat (triglycerides)**

| Sample | Heat treatment PI | Starch content | Viscosity in cp after 1 day in fridge (stdev) | Viscosity in cp after 3 weeks in fridge (stdev) |
|---|---|---|---|---|
| 2 (PI) | No | 1.75 | 53500 (9200) | 53000 (2200) |
| 4 (PI) | 30 min 80°C | 1 | 69000 (5700) | 61500 (2100) |
| 5 (ref, egg) | No | 2.5 | 50500 (3500) | 44500 (700) |
| 7 (ref, total protein) | No | 1.75 | 60000 (2800) | 64000 (5600) |
| 8 (PI) | No (only powder) | 1.75 | 49000 (1414) | 53500 (2121) |
| 9 (PI) | No | 1.75 | 52500 (2121) | 54500 (2121) |

The results illustrate that egg-protein is less effective as a viscosity enhancing protein: In order to obtain about the same viscosity, considerably more starch needs to be added. It is also shown that the viscosity-stability of the dressing containing egg-protein (sample 5) is considerably less: a decrease of 12 %, compared to 1 % only for sample 2.

The results for sample 4 and 8 show that heat treatment of the aqueous phase comprising the PI has a viscosity increasing effect on the final product. Thus, heat treatment can be used to increase the viscosity, whilst requiring less additional thickening agent.

**Table 3. Viscosity of dressing , containing 30 % fat (triglycerides)**

| Sample | Heat treatment PI | Starch content | Viscosity in cp after 1 day in fridge (stdev) | Viscosity in cp after 3 weeks in fridge (stdev) |
|---|---|---|---|---|
| 1 (PI) | No | 4.5 | 40500 (707) | 48000 (-) |
| 3 (PI) | 30 min 80°C | 4 | 48000 (5700) | 48000(15000) |
| 6 (ref, egg) | No | 5 | 34000 (-) | 31000 (2800) |

The results show that a heat treatment of the aqueous phase comprising PI results in a product with excellent viscosity-stability.

It is further shown that in this formulation (30 % fat) the target viscosity for this application is not reached with egg yolk (sample 6), despite a higher starch content and a three times higher protein content than in sample 1.

With respect to appearance: all dressings appeared shiny and stable (no phase separation) except for dressing 7 (ref), for at least 8 weeks after preparation. Dressing 7 became unstable between 3 and 8 weeks after preparation.

Regarding taste, the dressings had a satisfactory taste without any astringent sensation.

### Droplet size analysis

Droplet size of the dressings 1-5 was evaluated by microscope and Fritsch laser particle sizer (analysette 22 Compact), Idar-Oberstein, Germany) in a solution of EDTA (0.375% and Tween 20 (0.125%) at pH 10 (with NaOH). Around 0.3 g of dressing was dissolved in 30 ml of this solution. Next the solution was measured with the Fritsch.

Roughly all dressings show a peak at around 20 um with a shoulder around 1.5-2 um. Sample 4 showed a peak at around 15 um and a shoulder at 1.5 um and sample 5 showed a peak at 2.5 um and one at 20 um. The microscope showed similar ranges, however it is difficult to judge if there are any differences between the dressings.

### Satiety enhancing activity

The residual trypsin inihbition activity (TIA) and chymotrypsin inhibition activity (CTIA) for several dressings was determined. This is a measure for the presence of a satiety enhancing activity.

The activity was determined using the following azocaseine based assay and extraction of the reaction products of the protease activity was used:
A 30 mg / mL solution of azocasein (Sigma Aldrich, A2756) was prepared in prewarmed (60°C) 50 mM Tris / HCl buffer of pH 8.1 containing 100 mM of NaCl (Merck, 1.06404) and cooled down to 37°C. A series of 125 µL aliqouts containing increasing concentrations of the sample of interest were prepared for every sample. Typically, each 125 µL aliquot contained 1, 2, 3, 4 or 5 milligram of sample. Positive controls were performed by using 125 µL aliqouts containing only the buffer solution. To each of these aliqouts, 25 µL of a 400 µg/mL solution of trypsin (Fluka 93610) was added. For every sample, a blank measurement was performed by adding 25 µL of demineralised water rather than a protease solution. The blank measurements corresponding to the positive controls were used as negative controls. The resulting samples were thouroughly mixed by vortexing and incubated at 37°C for 10 minutes. To all samples, 225 µL aliquots of the azocasein solution were added, followed by an equally vigourous mixing step. These samples were incubated for 30 minutes exactly at 37°C upon which the proteolytic reaction was stopped by the addition of 125 µL of a 15% (w:v) solution of trichloroacetic acid (TCA) (Sigma-Aldrich, T9159). Undigested material was pelleted by centrifugation (10 minutes, 13.000 rpm, RT) and 100 µL of the supernatant of each sample was transferred to a 96-well microtiter plate. Proper solubilisation of the chromophoric peptides released by the protease from azocasein was ensured by the addition of 100 µL of a 1M solution of NaOH (VWR International, 28248.367). Absorbences at 440 nm were read automatically on a Model 680 microplate reader (BioRad). These absorbences are a measure for the quantity of peptides released from azocasein and hence for the amount of azocasein digested by the protease. The difference in A₄₄₀ between the positive and negative controls represents the amount of azocasein that is digested when no protease inhibitor is present, while the differences between samples and their sample blanks represents the amount of azocasein that is digested when protease inhibitors are present. Because both the constant amount of protease introduced in the system and the variable amounts of sample are known, the loss of proteolytic activity upon addition of the sample can be determined through lineair regression. This loss of proteolytic activity was expressed in mg of protease inhibited per gram of sample. For CTIA analysis, the procedure is essentially similar, but instead of a trypsin solution, a 800 µg / mL solution of chymotrypsin (Fluka 27270) and a 60 mg / mL solution of azocasein were used.

**Table 4: The residual TIA and CTIA activity in cold and heated dressings. Sample codes refer to recipes described in Table 1**

| Sample | % oil | %PI | % Egg yolk* powder | TIA (mg/g) | StdErr | CTIA (mg/g) | StdErr |
|---|---|---|---|---|---|---|---|
| 1 | 30 | 0.5 | 0 | 0.87 | 0.07 | 1.69 | 0.15 |
| 2 | 50 | 1.0 | 0 | 1.03 | 0.13 | 4.4 | 1.1 |
| 3 | 30 | 0.5 | 0 | 0.236 | 0.013 | 0.64 | 0.06 |
| 4 | 50 | 1.0 | 0 | 0.485 | 0.031 | 0.13 | 0.16 |
| 6 (ref) | 30 | 0 | 1.5 | 0.027 | 0.018 | 0.09 | 0.06 |

It is shown that the dressings comprising PI have a higher TIA and CTIA activity than the dressing without PI (sample 6).The heat stable PI fraction classified as PI-2 contains 41% stable form expressed as CTIA activity remaining after a heat treatment for 10 minutes at 80 ° C.

A solution of 5 g/l PI isolate yields 5.0 mg/ml equivalent TIA activity and 4.0 mg/ml equivalent CTIA activity. Homogenisation results in more than 40% inactivation outside the oil droplets or encapsulation of TIA and CTIA in the emulsion droplets. By encapsulation the proteinis less accessible for the inhibition assay. A temperature treatment of 10-30 minutes at 80 ° C and pH 3.0 results in the heat stable PI-2 activity of 1.25 mg/l and a CTIA equivalent of 2.0 g/l in the final product. In the heated emulsions with tested both emulsification and a heat treatment give a reduction of CTIA activity. Overall a PI-activity yield of 16% or more is found. Effective to be used for the delivery of PI-2 activity in these dressings.

### Example 2: Dressings prepared with a hot process

For the hot process a different starch was used (Eliane VE580, Avebe). Again Nive egg yolk powder was used as a first reference (minimal protein content of 30%) and a wheat protein Meripro 410 from Syral (80% protein), as a second reference. The same procedure as with the cold process of Example 1 was followed, only the water phase was heated before emulsification. The water phase included all ingredients except for the salts, the acids and the oil in the case of a dressing with PI and all ingredients except for egg, the acids and the oil in the case of a dressing with egg and all ingredients except for the acids and the oil in the case of a dressing with Meripro 410. This mixture is called a kuli. The kuli was heated to 85°C in a Stephan cooker and kept there for 5 minutes (this took around 1 hour). Next the kuli was cooled and added to the Fryma and the same procedure was followed as with the cold process. The dressing was then prepared in 2:40 minutes and filled into bags or cups.

**Table 5: Recipe of dressings as prepared by a hot process**

| Sample | 11 | 12 | 13 | 14 | 15 | 16 | 17 |
|---|---|---|---|---|---|---|---|
| Oil (soy oil) | 50 | 50 | 30 | 30 | 50 | 50 | 50 |
| Water | 33.45 | 35.85 | 53.15 | 54.75 | 34.05 | 35.8 | 35.6 |
| Egg yolk powder (Nive) | 2.5 | | 1.5 | | 2.5 | | |
| PI (S00756) | | 1 | | 0.5 | | 0.75 | |
| Meripro 410 (Syral) | | | | | | | 0.85 |
| Eliane VE580 | 2 | 1.1 | 3.3 | 2.7 | 1.4 | 1.4 | 1.5 |
| Salt | 0.6 | 0.6 | 0.6 | 0.6 | 0.6 | 0.6 | 0.6 |
| Dyon musterd | 2 | 2 | 2 | 2 | 2 | 2 | 2 |
| Sugar | 3 | 3 | 3 | 3 | 3 | 3 | 3 |
| Vinegar (Burg) | 3.55 | 3.55 | 3.55 | 3.55 | 3.55 | 3.55 | 3.55 |
| Lemon juice | 2.8 | 2.8 | 2.8 | 2.8 | 2.8 | 2.8 | 2.8 |
| K-sorbate | 0.1 | 0.1 | 0.1 | 0.1 | 0.1 | 0.1 | 0.1 |

### Results dressings hot preparation

Table 5 shows the recipes that were used for the preparation of dressings according the hot process. Another type of starch was used here (Eliane VE580) which was cooked together with the water phase. It should be noted that the salt was added afterwards for the dressings with PI, because PI is sensitive to salt and then gels too early. After storing the kuli with PI and salt overnight in the fridge emulsifying capacity was lost, whereby the kuli became essentially unsuitable for preparing a good dressing. All dressings appeared stable for at least several days and were very white. The viscosity was measured after 1 day and after 3 weeks. The results are shown in Table 6. Again the results show that PI contributes to the viscosity of the dressing more than egg does. Less starch is necessary to obtain a similar viscosity. The viscosity of the dressings with egg decreases a little bit in time. For dressings with PI the opposite is shown. This may have to do with the sensitivity of the starch for acid. With egg, the starch was added in the water phase which had a high pH. With the PI dressings the starch was added to a water phase that already had a low pH. It is also possible to prepare a 50% oil dressing including a heat treatment with only 0.75% PI. It is also possible to prepare a dressing with Meripro 410, however this dressing had a very low viscosity and there were some fish eyes visible already 1 day after preparation.

**Table 6: viscosities of dressings prepared according table 6 and as measured with a Brookfield viscometer**

| Sample | Heat treatment PI | Starch content | Viscosity in cp after 1 day in fridge (stdev) | Viscosity in cp after 3 weeks in fridge (stdev) |
|---|---|---|---|---|
| 11^{*} | Egg | 2 | 66000 (2828) | 61000 (1414) |
| 12 | 5 min 85°C (1% PI) | 1.1 | 58500 (7778) | 70400 (3394) |
| 13 | Egg | 3.3 | 18800 (283) | 17900 (141) |
| 14 | 5 min 85°C (0,5% PI) | 2.7 | 27800 (566) | 30400 (566) |
| 15 | Egg | 1.4 | 34400 (849) | 29900 (2121) |
| 16 | 5 min 85°C (0.75% PI) | 1.4 | 81000 (2828) | 91700 (424) |

| | | | | |
|---|---|---|---|---|
| *Kuli prepared day before and stored overnight in fridge (∼5°C) | | | | |

### Example 3: coacervates

### Introduction

Several emulsions were prepared comprising coacervates of potato protein protease inhibitor isolate (LMW-PPI), a high molecular weight potato protein isolate (HMW-PPI) containing predominantly patatines and whey protein isolate (WPI) respectively.

### Stability at pH 4, pH 7, 50 °C and 80 °C (Phase 1)

Experiments with respect to coacervation were aimed at the issue of stability at neutral pH. First a limited set of coacervates of LMW-PPI , HMW-PPI or WPI with a pectin were prepared at pH 4.

The coacervates (prepared at pH 4) were used as emulsifiers for oil-in-water emulsions and analysed for stability against creaming (formation of an oil layer on top) at pH 4 and increased pH 7, at room temperature, 50 °C and 80 °C.

### Simulation of physiological digestion of coacervate (Phase 2)

For these experiments, NIZO SIMPHYD (SIMulation of PHYsiological Digestion) which simulates the conditions of human digestion in vitro, was used.

### Materials

### Ingredients

The following ingredients were used:
Native potato protein isolates (Protease inhibitor isolate (LMW-PPI), 306 P7021, patatine isolate (HMW-PPI) 206 P7006 from
Solanic B.V LMW-PPI: potato protein isolate with a popotato protein protease inhibitor content >85 wt. %
Whey protein isolate (WPI, Bipro)
Low methylated (LM-)pectin (GENU® pectin type LM-12-CG) from CPKelco, Denmark
High methylated (HM-)pectin (CPKelco, 'Test sample no. 1795') Sunflower oil Reddy®, bought from supermarket

### Equipment

The following equipment was used:
Ultraturrax Polytron® PT3100 (Kinematica, Switserland) Homogenizer NS1001L2K (NIRO Soavi, Italy)

### Methods

### Coacervation between potato protein isolates and charged polysaccharides (Phase 1)

Protease inhibitor isolate (LMW-PPI), patatin protein isolate (HMW-PPI) and whey protein isolate (WPI) solutions were made by dissolving 1 wt.% of protein powder in demineralized water on a magnetic stirrer. Polysaccharide solutions were made (1wt.% Na-alginate, 1wt.% gum Arabic, 1wt.% LM-pectin and 1wt.% HM-pectin). Further, 0.4 wt.% gelatin was made. The protein solution was added to the polysaccharide or gelatin solution at a 1:1 volume ratio and pH was adjusted with 0.5 N HCl or 0.5 N NaOH.

### Preparation of coacervate oil in water emulsions (Phase 1)

20 wt.% sunflower oil emulsions were made with 0.25 wt.% protein (m/m) and 0.5 wt.% polysaccharide/gelatin solution. Therefore an emulsion of 320 g oil, 400 g 1% protein (m/m) and 80 g water was mixed with an ultraturrax at 25 000 rpm and homogenized at 150/20 bar. The protein/oil emulsion was added to the polysaccharide solution at a 1:1 volume ratio. The final solution contains 0.25% protein.

### Simulation of the gastro-intestinal tract (SIMPHYD) (Phase 2)

The exposure to the simulated conditions (pH and enzymes) of the gastro-intestinal tract involved the following steps:
- Adjust pH to 2.0 using HCl
- Visually determine possible instability
- Add gastric juice (pepsin)
- Incubate at 37 °C
- Every 15 min visually determine instability
- After 2h adjust pH to 6.0 using NaHCO₃
- Visually determine possible instability
- Add pancreatic solution
- Incubate 1.5 h 37 °C
- Every 15 min visually determine instability

The reactions were carried out in an Erlenmeyer in a shaking water bath. After addition of gastric juice or pancreatic solution and stirring a small sample was taken for evaluation of the stability. This small sample was added to a test tube and no longer stirred during incubation.

Microscopic images were made after incubation with pancreatic solution.

### Results and discussion

### Stability of emulsion at different pH and after heating

Emulsion stability was determined by visual observation. The appearance of a dense layer on top of a more transparent layer indicates creaming. The thicker the layer, the less stable is the emulsion.

It was found that LMW-PPI coacervates had a better stabilising effect on the emulsion than HMW-PPI coacervates, at pH 4 and at pH 7. The emulsions with LM-pectin coacervate appeared more stable than with HM-pectin, at pH 7 (in the emulsions comprising HM-pectin large oil droplets were formed in the top layer after a few days. This was not observed in case of LM-pectin. By definition, preparations in which more than half of the carboxyl groups are in the methyl ester form (-COOCH3) are classified as high methoxyl (HM) pectins; the remainder of the carboxyl groups will be present as a mixture of free acid (-COOH) and salt (-COO-Na⁺) forms. Preparations in which less than half of the carboxyl groups are in the methyl ester form are called low-methoxyl (LM) pectins (Damodoran, S., Parkin, K.L., Fennema, O.R., (2008), Fennema's Food Chemistry, CRC Press, Boca Raton, USA.).

The stabilizing protein has a strong influence on the effect of heating for 1 h at 50 or 80 °C.

The emulsions comprising coacervate of WPI or HMW-PPI with and LM-pectin showed considerable phase separation after the heat treatment. The heat stability of a coacervate of HMW-PPI with HM-pectin was also poor. No results were obtained for WPI with HM-pectin.

The emulsions comprising a coacervate of LM-pectin or HM-pectin with LMW-PPI , however, appeared (nearly) completely without phase separation after the heat treatment at 50 or 80 °C.

### Stability of coacervates in simulated physiological model

In order to test the possible advantages of LMW-PPI for emulsion stability in the gastro-intestinal tract, two emulsions, one stabilized by WPI/LM-pectin, and the other by LMW-PPI/LM-pectin (20 wt. % sunflower emulsion, as described above), were exposed to a sequence of conditions (SIMPHYD, see above) typical of the upper gastro-intestinal digestion.

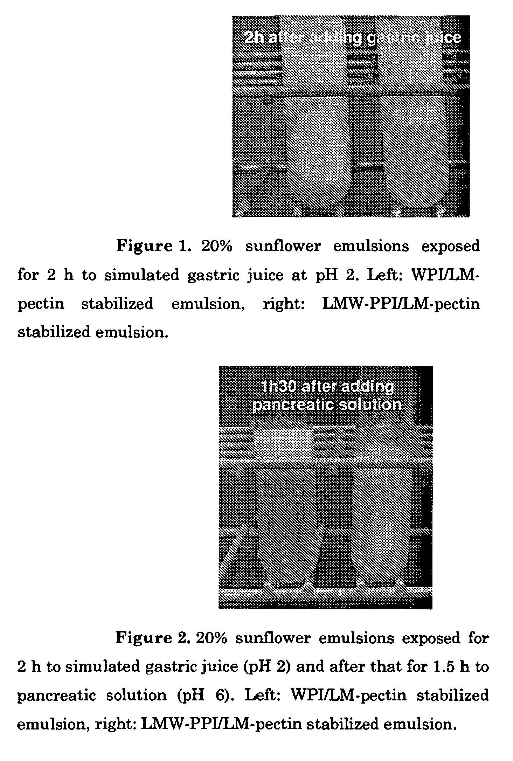

Exposure to gastric juice (pH 2, 2hr) destabilized both emulsions of WPI and LMW-PPI (Figure 1), but more so in the case of LMW-PPI.

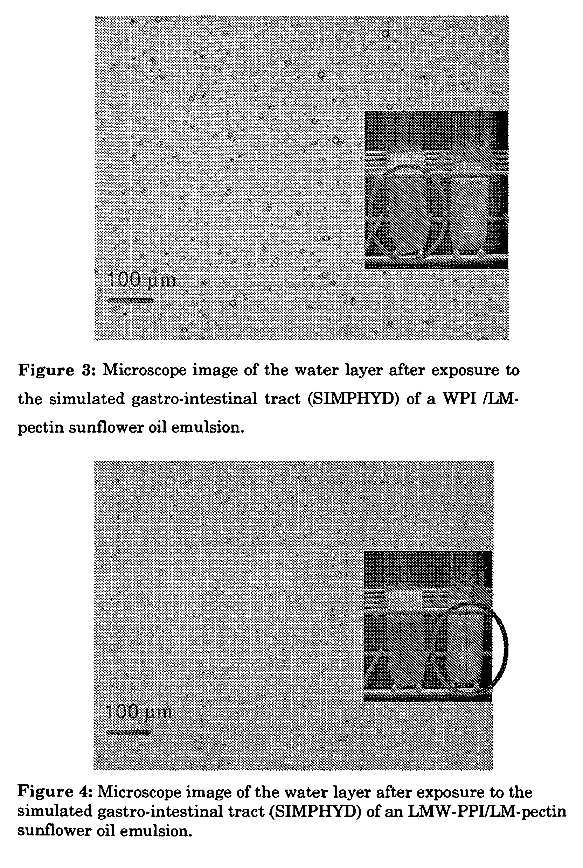

Subsequent exposure to pancreatic solution (pH 6, 1.5 hr) caused further destabilization of the emulsion and a thick layer of oil was formed (Figure 2, top layer). Based on the volume of this layer, it is expected that this layer contains nearly all the oil originally emulsified. However, the final turbidity of the water layer is much higher in the case of LMW-PPI, suggesting that at least a part of the oil is still in a stable emulsion. Inspection by microscopy (Figure 3 and Figure 4) confirms that the turbidity is at least partly due to small oil droplets. In the case of WPI these oil droplets are larger, corresponding to a lower stability. This is an indication that a part of the original emulsion is retained in a more stable state by LMW-PPI than by WPI. This supports the inventors' insight that PPI coacervates , may offer an advantage in order to stabilize oil during gastro-intestinal passage, possibly up to the ileum thereby creating possibilities for formulating a condiment or other food product which may trigger or contribute to triggering the ileal break mechanism and/or may be used in a diet for preventing or treating overweight.

### Example 4: freeze-thaw stability

### Materials and methods.

### Cold process

Dressings were prepared with a Fryma colloid mill, in a similar way as in Example 1, with the proviso that the polysaccharides were added as indicated below. The potato protein protease inhibitor isolatewas Solanic® 306p.

In total 6 kg of dressing was prepared per batch.. Soy oil from Romy was used. The lemon juice and the vinegar were added at the end. The polysaccharides were added together with the acids, after the emulsion was prepared (roughly half way the process). The Fryma was operated for 3 minutes per batch in total (including adding the oil). Table 6 shows the recipes that were tested.

**Table 7: Recipe of 50% oil dressings**

| Recipe | **1** | **2** | **3** | **4** | **5** | **6** | **7** | **8** |
|---|---|---|---|---|---|---|---|---|
| oil (soy oil) | 50 | 50 | 50 | 50 | 50 | 50 | 50 | 50 |
| Nive egg yolk powder | | | | | 2.5 | 2.5 | | |
| PI | 1 | 1 | 1 | 1 | | | | |
| waxy potato starch (Eliane SC 160) | 1.5 | 0 | 0 | 0 | 1 | 2.5 | 1 | 2.5 |
| octenyl succinic acid amylopectin starch Eliane MC160 | | | | | | | 1 | 1 |
| Water | 35.45 | 36.75 | 36.75 | 36,75 | 34.45 | 32.75 | 35.95 | 34.25 |
| Vinegar | 3.55 | 3.55 | 3.55 | 3,55 | 3.55 | 3.55 | 3.55 | 3.55 |
| Lemon juice | 2.8 | 2.8 | 2.8 | 2,8 | 2.8 | 2.8 | 2.8 | 2.8 |
| Dyon mosterd | 2 | 2 | 2 | 2 | 2 | 2 | 2 | 2 |
| Sugar | 3 | 3 | 3 | 3 | 3 | 3 | 3 | 3 |
| Salt | 0.6 | 0.6 | 0.6 | 0,6 | 0.6 | 0.6 | 0.6 | 0.6 |
| K sorbate | 0.1 | 0.1 | 0.1 | 0,1 | 0.1 | 0.1 | 0.1 | 0.1 |
| LM-pectine (LM-12-CG from CPKelco) | | 0.2 | | | 0.2 | | 0.2 | |
| HM-pectine (YM-115-L from CPKelco) | | | 0.2 | | | | | |
| Xanthan (Keltrol F CPKelco) | | | | 0.2 | | | | |

| | | | | | | | | |
|---|---|---|---|---|---|---|---|---|
| Abbreviations are as indicated above, unless specified otherwise | | | | | | | | |

### Storage

The dressings were frozen for 1 night at -20°C and thawed at room temperature. The dressings comprising egg yolk (5,6)were not stable. The dressings with Eliane MC 160 (7,8) showed some oil on top.

After a second freeze-thaw cycle (1 night at -20°C), the phase separation for dressings 7,8 was more pronounced, whilst the dressing comprising both the potato protein protease inhibitor and the hydrocolloid (pectin for dressings 2 and 3, xanthan gum for dressing 4) did not show any oil phase separation or water phase separation. Of these dressings dressing 2 (potato protein protease inhibitor and LM pectin) had the smoothest structure.

### Texture measurements with Brookfield

The viscosity of the dressings was evaluated with a Brookfield viscometer, as described above. The viscosity was measured prior to freezing, 1 day after preparation of the dressings. The results are as shown in Table 7:.

**Table 8:**

| Dressing | Viscosity after 1 day (cp) | standard deviation |
|---|---|---|
| 1 | 26800 | 566 |
| 2 | 22000 | 849 |
| 3 | 53500 | 2121 |
| 4 | 5900 | 1273 |
| 5 | 29700 | 990 |
| 6 | 51700 | 4384 |
| 7 | 29800 | 1131 |
| 8 | 56200 | 283 |

## Claims

1. Condiment comprising an emulsion of an aqueous phase and an oil or fat phase, the emulsion comprising a potato protein protease inhibitor isolate, wherein the condiment further comprises at least one ingredient selected from the group of thickening agents, food acids, flavourings, preservatives, anti-oxidants and colourings.

2. Condiment according to claim 1, wherein the potato protein protease inhibitor isolate is present in a concentration of 0.1 to 4 wt. %, in particular in a concentration of 0.2-2.0 wt. %, more in particular in a concentration of 0.5 to 1.0 wt. %.

3. Condiment according to claim 1 or 2, wherein the fraction of potato protein protease inhibitor relative to the total protein content in the emulsion is at least 50 wt. %, in particular at least 75 wt. %, more in particular at least 85 wt. %.

4. Condiment according to any of the preceding claims, wherein at least a part of the of potato protein protease inhibitor is present as a coacervate of the potato protein protease inhibitor and a hydrocolloid, which hydrocolloid may in particular be selected from the group of pectins, arabic gum, alginates and gelatines.

5. Condiment according to any of the preceding claims, wherein the pH of the water phase of the condiment as measured by a pH meter, is in the range of 2.5-6.5 in particular in the range of 3.2-4.2.

6. Condiment according to any of the preceding claims, wherein the oil content is 3 to 80 wt. %, based on the total weight, in particular 15 to 60 wt. %, based on the total weight, more in particular 25 to 50 wt. %, based on the total weight.

7. Condiment according to any of the preceding claims wherein the oil or fat phase is a vegetable oil or fat phase.

8. Condiment according to any of the preceding claims, having a viscosity as measured with a Brookfield type viscometer at a temperature of 5°C with spindle S93 and a speed of 10 rpm in the range of 8000 cp to 180000 cp, in particular in the range of 25000 cp to 140000 cp , more particular in the range of 35000 cp to 45000 cp.

9. Condiment according to any or the preceding claims which is essentially free of gluten, egg proteins, dairy proteins and egg-yolk.

10. Condiment according to any of the preceding claims, wherein the condiment is selected from the group of spreads and dressings, in particular from the group of mayonnaises, mayonnaise-substitutes, salad dressings and Hollandaise sauce.

11. Condiment according to any of the preceding claims, wherein the condiment is essentially free of artificial emulsifiers, such as chemically derivatised emulsifying polysaccharides.

12. Method for preparing a condiment according to any of the preceding claims, comprising preparing an aqueous phase comprising the protein protease inhibitor isolate and thereafter dispersing the oil or fat phase in the aqueous phase or dispersing the aqueous phase in the oil or fat phase; and adding an ingredient selected from the group of thickening agents, food acids, flavourings, preservatives, anti-oxidants and colourings to the aqueous phase or the oil or fat phase prior to dispersion, or thereafter to the emulsion.

13. Method according to claim 12, wherein an aqueous phase comprising the protein protease inhibitor isolate is subjected to a heat treatment, in particular a heat treatment at a temperature in the range of 50-85 °C, more in particular in the range of 65-85 °C or 75-85 °C.

## Patentansprüche

1. Würzmittel, umfassend eine Emulsion einer wässrigen Phase und einer Öl- oder Fettphase, die Emulsion umfassend ein Kartoffelproteinprotease-Inhibitorisolat, wobei das Würzmittel weiterhin mindestens eine Zutat, ausgewählt aus der Gruppe von Dickungsmitteln, Lebensmittelsäuren, Aromastoffen, Konservierungsstoffen, Antioxidanzien und Farbstoffen, umfasst.

2. Würzmittel nach Anspruch 1, wobei das Kartoffelproteinprotease-Inhibitorisolat in einer Konzentration von 0,1 bis 4 Gew.-%, insbesondere in einer Konzentration von 0,2-2,0 Gew.-%, mehr im Besonderen in einer Konzentration von 0,5 bis 1,0 Gew.-% vorhanden ist.

3. Würzmittel nach Anspruch 1 oder 2, wobei der Anteil vom Kartoffelproteinprotease-Inhibitor in Bezug auf den Gesamtproteingehalt in der Emulsion mindestens 50 Gew.-%, insbesondere mindestens 75 Gew.-%, mehr im Besonderen mindestens 85 Gew.-% beträgt.

4. Würzmittel nach einem der vorhergehenden Ansprüche, wobei mindestens ein Teil des Kartoffelproteinprotease-Inhibitors als ein Koazervat des Kartoffelproteinprotease-Inhibitors und eines Hydrokolloids, wobei das Hydrokolloid insbesondere aus der Gruppe von Pektinen, Gummiarabikum, Alginaten und Gelatinen ausgewählt werden kann, vorhanden ist.

5. Würzmittel nach einem der vorhergehenden Ansprüche, wobei der pH der Wasserphase des Würzmittels, wie mit einem pH-Meter gemessen, in dem Bereich von 2,5-6,5, insbesondere in dem Bereich von 3,2-4,2, liegt.

6. Würzmittel nach einem der vorhergehenden Ansprüche, wobei der Ölgehalt 3 bis 80 Gew.-%, basierend auf dem Gesamtgewicht, insbesondere 15 bis 60 Gew.-%, basierend auf dem Gesamtgewicht, mehr im Besonderen 25 bis 50 Gew.-%, basierend auf dem Gesamtgewicht, beträgt.

7. Würzmittel nach einem der vorhergehenden Ansprüche, wobei die Öl- oder Fettphase eine pflanzliche Öl- oder Fettphase ist.

8. Würzmittel nach einem der vorhergehenden Ansprüche, mit einer Viskosität, wie gemessen mit einem Viskosimeter vom Typ Brookfield, bei einer Temperatur von 5 °C mit Spindel S93 und einer Drehzahl von 10 U/Min. in dem Bereich von 8000 cP bis 180000 cP, insbesondere in dem Bereich von 25000 cP bis 14000 cP, mehr im Besonderen in dem Bereich von 35000 bis 45000 cP.

9. Würzmittel nach einem der vorhergehenden Ansprüche, das im Wesentlichen frei von Gluten, Eiproteinen, Milchproteinen und Eigelb ist.

10. Würzmittel nach einem der vorhergehenden Ansprüche, wobei das Würzmittel aus der Gruppe von Aufstrichen und Saucen, insbesondere aus der Gruppe von Mayonnaisen, Mayonnaise-Ersatzstoffen, Salatsaucen und Sauce Hollandaise, ausgewählt ist.

11. Würzmittel nach einem der vorhergehenden Ansprüche, wobei das Würzmittel im Wesentlichen frei von künstlichen Emulgatoren wie etwa chemisch abgeleiteten emulgierenden Polysacchariden ist.

12. Verfahren zur Herstellung eines Würzmittels nach einem der vorhergehenden Ansprüche, umfassend Herstellen einer wässrigen Phase, umfassend das Proteinprotease-Inhibitorisolat, und anschließend Dispergieren der Öl- oder Fettphase in der wässrigen Phase oder Dispergieren der wässrigen Phase in der Öl- oder Fettphase; und Hinzufügen einer Zutat, ausgewählt aus der Gruppe von Dickungsmitteln, Lebensmittelsäuren, Aromastoffen, Konservierungsstoffen, Antioxidanzien und Farbstoffen zu der wässrigen Phase oder der Öl- oder Fettphase vor dem Dispergieren, oder danach zu der Emulsion.

13. Verfahren nach Anspruch 12, wobei eine wässrige Phase, umfassend das Proteinprotease-Inhibitorisolat einer Wärmebehandlung unterzogen wird, insbesondere einer Wärmebehandlung bei einer Temperatur in dem Bereich von 50-85 °C, mehr im Besonderen in dem Bereich von 65-85 °C oder 75-85 °C.

## Revendications

1. Condiment comprenant une émulsion d'une phase aqueuse et d'une phase huileuse ou de matière grasse, l'émulsion comprenant un isolat d'inhibiteur de protéase de protéine de pomme de terre, dans lequel le condiment comprend en outre au moins un ingrédient sélectionné dans le groupe comprenant les agents épaississants, les acides alimentaires, les aromatisants, les conservateurs, les antioxydants et les agents colorants.

2. Condiment selon la revendication 1, dans lequel l'isolat d'inhibiteur de protéase de protéine de pomme de terre est présent à une concentration de 0,1 à 4 % en poids, en particulier à une concentration de 0,2 à 2,0 % en poids, plus particulièrement à une concentration de 0,5 à 1,0 % en poids.

3. Condiment selon la revendication 1 ou 2, dans lequel la fraction d'inhibiteur de protéase de protéine de pomme de terre par rapport à la teneur totale en protéines dans l'émulsion est au moins de 50 % en poids, en particulier au moins de 75 % en poids, plus particulièrement au moins de 85 % en poids.

4. Condiment selon l'une quelconque des revendications précédentes, dans lequel au moins une partie de l'inhibiteur de protéase de protéine de pomme de terre est présente sous la forme d'un coacervat de l'inhibiteur de protéase de protéine de pomme de terre et d'un hydrocolloïde, ledit hydrocolloïde pouvant en particulier être sélectionné dans le groupe comprenant les pectines, la gomme arabique, les alginates et les gélatines.

5. Condiment selon l'une quelconque des revendications précédentes, dans lequel le pH de la phase aqueuse du condiment, tel que mesuré par un pH-mètre, est situé dans la plage allant de 2,5 à 6,5, en particulier dans la plage allant de 3,2 à 4,2.

6. Condiment selon l'une quelconque des revendications précédentes, dans lequel la teneur en huile est de 3 % à 80 % en poids sur la base du poids total, en particulier de 15 % à 60 % en poids sur la base du poids total, plus particulièrement de 25 % à 50 % en poids sur la base du poids total.

7. Condiment selon l'une quelconque des revendications précédentes, dans lequel la phase huileuse ou de matière grasse est une huile végétale ou une phase de matière grasse.

8. Condiment selon l'une quelconque des revendications précédentes, présentant une viscosité, telle que mesurée avec un viscosimètre de type Brookfield à une température de 5 °C avec une tige S93 et une vitesse de 10 tours/minute, située dans la plage allant de 8 000 cp à 180 000 cp, en particulier dans la plage allant de 25 000 cp à 140 000 cp, plus particulièrement dans la plage allant de 35 000 cp à 45 000 cp.

9. Condiment selon l'une quelconque des revendications précédentes, qui est essentiellement exempt de gluten, de protéines d'oeuf, de protéines de lait et de jaune d'oeuf.

10. Condiment selon l'une quelconque des revendications précédentes, dans lequel le condiment est sélectionné dans le groupe comprenant les produits à tartiner et les sauces, en particulier dans le groupe comprenant les mayonnaises, les substituts de mayonnaise, les vinaigrettes et la sauce hollandaise.

11. Condiment selon l'une quelconque des revendications précédentes, dans lequel le condiment est essentiellement exempt d'émulsifiant artificiel, tel que les émulsifiants polysaccharidiques chimiquement dérivatisés.

12. Méthode de préparation d'un condiment selon l'une quelconque des revendications précédentes, comprenant la préparation d'une phase aqueuse comprenant l'isolat d'inhibiteur de protéase de protéine et ensuite la dispersion de la phase huileuse ou de matière grasse dans la phase aqueuse ou la dispersion de la phase aqueuse dans la phase huileuse ou de matière grasse ; et l'ajout d'un ingrédient sélectionné dans le groupe comprenant les agents épaississants, les acides alimentaires, les aromatisants, les conservateurs, les antioxydants et les agents colorants à la phase aqueuse ou la phase huileuse ou de matière grasse avant la dispersion, ou par la suite à l'émulsion.

13. Méthode selon la revendication 12, dans laquelle une phase aqueuse comprenant l'isolat d'inhibiteur de protéase de protéine est soumise à un traitement thermique, en particulier un traitement thermique à une température située dans la plage allant de 50 °C à 85 °C, plus particulièrement dans la plage allant de 65 °C à 85 °C ou de 75 °C à 85 °C.
